# EUROPEAN PATENT APPLICATION

(11) **EP 1 880 706 A1**
(43) Date of publication of application: **23.01.2008**
(21) Application number: 06015214.7
(22) Date of filing: 21.07.2006
(51) Int. Cl.: A61K 8/44, A61Q 5/04

(54) **Method and composition for permanently shaping hair**

(71) Applicant: Wella Aktiengesellschaft, 64274 Darmstadt (DE)
(72) Inventor: Cassier, Thorsten, Dr., 64807 Dieburg (DE); Schreiber, Birgit, 64678 Lindenfels (DE); Steinbrecht, Karin, Dr., 64372 Ober-Ramstadt (DE)

(57) **Abstract**

A composition for permanent hair shaping comprising
(i) 0.5 to 35 % by weight of a N-alkyl-2-mercaptoacetamide of formula 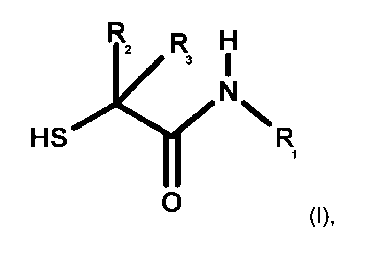 wherein R₁ is a straight chain alkyl residue with 3 to 6 carbon atoms or a straight chain hydroxyalkyl with 3 to 6 carbon atoms, R₂ and R₃ are, independently from each other, hydrogen or straight chain alkyl residues with 1 to 3 carbon atoms, or the salt thereof,
(ii) 0.1 to 15 % by weight of at least one cationic polymer and
(iii) 5 to 95 % by weight of water; and a method of permanently shaping hair using said composition.

## Description

### Field of the Invention

The object of the present invention is a composition on the basis of a combination of N-alkyl-2-mercaptoacetamide and a cationic polymer for permanently shaping hair and a process therefore.

### Prior Art

The classic technique for permanent hair waving is familiarly based on two treatment steps: In the first step, the cystine-disulfide bridges of the keratin of the hair are opened by the action of an agent that contains a reducing ingredient (waving composition). The hair is then put into the desired form. In a second step, cystine-disulfide bonds are closed again using a fixative, that is, an agent containing an oxidant ingredient.

Thioglycolic acid, for instance in the form of an ammonium or monoethanolamine salt, is used as a classic permanent hair waving reducing agent. Other typical reducing agents are 2-mercaptopropionic acid (thiolactic acid), 3-mercaptopropionic acid, cysteine, and derivatives of these compounds, as well as certain mercaptocarboxylic acid esters.

Alkaline-adjusted preparations based on mercaptocarboxylic acid esters, despite being adequately effective, cause hair damage, which is expressed for instance in increased hair breakage. Often, these compositions also undesirably stress the scalp. Finally, the unpleasant odor of the reducing agents used requires intensive perfuming of the products. By using 2-mercaptopropionic acid, it is possible to solve some of these problems. However, in comparison to thioglycolic acid, which is widely used, thiolactic acid produces weaker waves.

For gentle permanent waving of damaged and especially bleached or dyed hair, waving compositions that are adjusted to be slightly acidic to neutral are preferably used. From a professional standpoint, over the last 35 years, the thioglycolic acid esters have proved to be the reducing agents best suited for this purpose.

A major disadvantage of acidic hair waving compositions based on thioglycolic acid esters, however, is that they are poorly tolerated by the eyes and skin, and the sensitizing effect of the thioglycolic acid esters, so that the use of these hair waving compositions is widely avoided at present.

In our own EP 0969791 B1 it is reported that N-(3'-hydroxypropyl)-2-mercaptoacetamide has substantially lower sensitization rates than their closest relatives in permanent waving ingredients, the N-alkyl-2-mercaptoacetamides known from the prior art DE 1 144 440 C and European Patent EP-A 0 455 457 A1, that is N-methylmercaptoacetamide and N-hydroxyethylmercaptoacetamide.

It is well known that the combination of known waving agents like thioglycolic acid, cysteamine, cysteine, thiolactic acid and others with cationic polymers improve the condition of the hair with respect to combability. Yet the problem of fading of the color of dyed hair and the bleeding out of the color could not be solved thereby sufficiently.

It is a therefore the main goal of the invention to provide a permanent shaping composition and method that delivers unique elasticity and strength of the shaped hair from the tip to the root, an improved uniformity of the shaped hair with less disordered appearance and which simultaneously prevents the dyed hair from fading of the color and the bleeding out of the color.

Thioglycolic acid glyceryl esters are superior to thioglycolic acid and its salts in this respect but unfortunately these esters display the disadvantage of being sensitizing to the scalp and the hands of the hair dresser. Thioglycolic acid and ist salts in combination with at least one cationic polymer on the other side does not sensitize the skin as much as the glyceryl thioglycolate but it does not leave the hair in such a good condition after waving as the ester does.

A further goal of the present invention is therefore to provide a composition and a method for permanent shaping hair which does not sensitize and irritate the skin and the scalp of man in a way that thioglycolic acid and its salts and particulary thioglycolic esters do.

A further goal of the present invention is to provide a composition and a method for permanent shaping hair which has superior hair care effects especially with regard to improved soft feel of wet and dry hair, improved elasticity of hair and durable waving of the hair.

Another goal of the present invention is to provide a composition and a method for permanent shaping hair which improves the odor of the treated hair significantly during and after the hair shaping process and which is superior to former compositions and thus enhances the customers acceptance of permanent hair shaping products.

Another aim of the present invention is to provide a composition and a method for permanent shaping hair which causes less hair damage than compositions of the

### prior art.

A further aim of the present invention is to provide a composition and a method for permanent shaping hair which penetrates well into the hair but less into the skin than compositions of the prior art.

The above problems are solved by a composition and a method as recited in claims 1 and 13 respectively.

The preferred embodiments are indicated in the sub-claims.

The first essential component of the permanent hair shaping composition, the N-alkyl-2-mercaptoacetamide of formula (I), is contained in the composition for permanent hair shaping hair in a quantity of from about 0.5% to about 35% by weight, preferably from about 3% to about 25% by weight and most preferred from about 4% to about 20% by weight of the composition. The preferred salts of N-alkyl-2-mercaptoacetamide of formula (I) are the chloride, sulfate, acetate or citrate.

The N-alkyl-2-mercaptoacetamide of formula (I), whereby N-(3'-hydroxypropyl)-2-mercaptoacetamide is preferred, is preferably contained as the sole keratin-reducing agent in the composition. However, it may also be present in combination with other keratin-reducing agents, such as thioglycolic acid, thiolactic acid, 2-hydroxy-3-mercaptopropionic acid, or the salt thereof; cysteamine and cysteamine derivatives such as alkyl- or acylcysteamine; further cysteine and cysteine derivatives, such as cysteine-(2-hydroxyethyl)ester and L-cysteine glycerine ester; or sulfites. If the N-alkyl-2-mercaptoacetamide of formula (I) is present in combination with other keratin-reducing agents, it is preferred that it is present in excess of the total amount of these other keratin-reducing agents.

The permanent shaping composition comprises the keratin-reducing agents in the quantities typical for shaping, for example the mixture of N-alkyl-2-mercapto-acetamide of formula (I) with the ammonium salts of thioglycolic acid, thiolactic acid or cysteine, in a total amount of from about 0.5% to about 35% by weight, preferably from about 2% to about 28% by weight and most preferred from about 4% to about 20% by weight of the composition.

It is preferred that the other hair keratin-reducing agents be a salt or the derivative of a mercapto carboxylic acid. It is especially preferred that the other keratin-reducing agents be selected from mercaptoacetic acid, cysteine, and thiolactic acid, or salts thereof.

The second essential component of the permanent hair shaping composition of the invention is a cationic copolymer, a cationic polysaccharide or a cationic silicon polymer, where the quaternary nitrogen groups are contained either in the polymer chain or preferably as substituents on one or more of the monomers. The ammonium group containing monomers can be copolymerized with non-cationic monomers. Suitable cationic monomers are unsaturated, radical polymerizable compounds carrying at least one cationic group, particularly ammonium-substituted vinyl monomers such as trialkyl methacryl oxyalkyl ammonium, trialkyl acryloxyalkyl ammonium, dialkyl diallyl ammonium, and quaternary vinyl ammonium monomers with groups containing cyclic, cationic nitrogen such as pyridinium, imidazolium, or quaternary, e.g. alkyl vinyl imidazolium, alkyl vinyl pyridinium, or alkyl vinyl pyrrolidone salts. The alkyl groups of these monomers are preferably lower alkyl groups, such as C1 to C7 alkyl groups, with C1 to C3 alkyl groups being especially preferred.

The ammonium group-containing monomers can be copolymerized with non-cationic monomers. Suitable co-monomers are, for example, acrylamide, methacrylamide, alkyl- and dialkyl acrylamide, alkyl- and dialkyl methacrylamide, alkyl acrylate, alkyl methacrylate, vinyl caprolactone, vinyl caprolactam, vinyl pyrrolidone, vinyl ester, e.g. vinyl acetate, vinyl alcohol, propylene glycol, or ethylene glycol, wherein the alkyl groups of these monomers are preferably C1 to C7 alkyl groups, with C1 to C3 alkyl groups being especially preferred.

Preferred polymers with quaternary amine groups are polymers selected from those described in the International Cosmetic Ingredient Dictionary an Handbook "CTFA" (10th Edition of 2004) under the designations as follows: the polymers sold by the company Rhodia Inc. under the trade names Mirapol^{®} A15 (POLYQUATERNIUM-2); the copolymer of hydroxyethyl cellulose and diallyldimethylammonium chloride (POLYQUATERNIUM-4); the copolymer of acrylamide and diallyldimethylammonium chloride, sold by the company Ondeo Nalco under the trade names Merquat^{®} 550, Merquat^{®} 550L and Merquat^{®} S (POLYQUATERNIUM-7); vinyl pyrrolidone/dimethylamino ethylmethacrylate methosulfate copolymer (POLYQUATERNIUM-11), sold under the trade names Gafquat^{®} 755 N and Gafquat^{®} 734 by GAF Corp. USA and under the trade name Luviquat^{®} PQ 11 PN by BASF/Germany; Gafquat® 734 is especially preferred; copolymer of acrylamide and betamethacrylyloxyethyl trimethyl ammonium chloride, sold by Rohm GmbH in Germany under the trade name Rohagit^{®} KF 720 (POLYQUATERNIUM-15); a copolymer sold by BASF in Germany under the trade name LUVIQUAT^{®} FC 550, LUVIQUAT^{®} FC905 and LUVIQUAT^{®} HM 552 consisting of polyvinyl pyrrolidone and imidazolimine methochloride (POLYQUATERNIUM-16); Mirapol^{®} AD1 (POLYQUATERNIUM-17), Mirapol^{®} AZ1 (POLYQUATERNIUM-1 8); the copolymer of diallyldimethyl ammonium chloride and acrylic acid (80/20), sold by the company Calgon under the trade name Merquat^{®} 280 (POLYQUATERNIUM-22); polymers sold under the trade name Quatrisoft^{®} LM 200 by the company Amerchol, defined as polymeric quaternary ammonium salt of hydroxyethyl cellulose reacted with a lauryl dimethyl ammonium substituted epoxide, sold under the trade name Mirapol^{®} 175 (POLYQUATERNIUM-24); copolymer of vinyl pyrrolidone and methacrylamidopropyl trimethyl ammonium chloride (POLYQUATERNIUM-28, CAS 131954-48-8), sold under the trade name Gafquat^{®} HS-100 by GAF Corp.; the copolymer of acrylamide and dimethylaminoethyl methacrylate (20/80 by weight) quaternized with methyl chloride, sold as a 50% by weight dispersion in a mineral oil under the trade name Salcare^{®} SC92 by the company Ciba Specialty Chemicals (POLYQUATERNIUM-32); the methosulfate of the copolymer of methacryloyloxyethyl trimethylammonium and methacryloyloxyethyl dimethylacetylammonium, sold under the trade name Plex^{®} 7525L and Plex^{®} 3074L by the company Rohm GmbH/Germany (POLYQUATERNIUM-35); a terpolymer sold by Calgon in the USA under the trade name Merquat^{®} Plus 3300 consisting of dimethyl diallyl ammonium chloride, sodium acrylate and acrylamide (POLYQUATERNIUM-39); a terpolymer from ISP in the USA, sold under the trade name Gaffix^{®} VC 713, consisting of vinyl pyrrolidone, dimethylamino ethyl methacrylate, and vinyl caprolactam; and the cationic polymer (POLYQUATERNIUM-67) sold under the trade name Polymer SL^{®}-60 by the company Amerchol.

### Cationic Polysaccharide

Suitable cationic polymers that are derived from natural polymers are cationic derivatives of polysaccharides, for example, cationic derivatives of cellulose, starch or guar. Furthermore, chitosan and chitosan derivatives are suitable. Cationic polysaccharides have the general formula

G-O-B-N⁺R^{a}R^{b}R^{c} · X⁻ (II)

G is an anhydroglucose residue, for example, starch or cellulose anhydroglucose; B is a divalent bonding group, for example alkylene, oxyalkylene, polyoxyalkylene or hydroxy-alkylene;
R^{a}, R^{b}, and R^{c}, independently from one another, are alkyl, aryl, alkylaryl, arylalkyl, alkoxyalkyl, or alkoxyaryl each having 1 to 18 C atoms, wherein the total number of C atoms in R^{a}, R^{b}, and R^{c} is preferably a maximum of 20;
X (-) is a common anion, preferably chloride, bromide, or sulfate.

A suitable cationic cellulose is sold by Amerchol under the name Polymer JR and has the INCI designation quaternized hydroxyethyl cellulose (CTFA: POLYQUATERNIUM-10). An additional cationic cellulose has the INCI designation POLYQUATERNIUM-24 and is sold by Amerchol under the trade name Polymer LM-200. A suitable cationic guar derivative is sold under the trade name Jaguar^{®} R and has the INCI designation GUAR HYDROXYPROPYLTRIMONIUM CHLORIDE.

Other suitable cationic polysaccharides are chitosan, chitosan salts, and chitosan derivatives. The chitosans are completely or partially de-acetylated chitins (POLYQUATERNIUM-29). To produce a chitosan, one preferably starts with the chitin contained in the shell residues of crustaceans, which, as a cheaper and natural raw material, is available in large quantities. The molecular weight of the chitosan can be distributed across a broad spectrum, for example, of from 20,000 to approx. 5 million g/mol (700 to about 176,000 oz/mol). For example, a suitable low-molecular-weight chitosan is one with a molecular weight of from 30,000 to 70,000 g/mol (1,100 to 25,000 oz/mol). Preferably, however, the molecular weight is greater than 100,000 g/mol (3,527 oz/mol), or especially preferred is a molecular weight of from 200,000 to 700,000 g/mol (7,054 to 24,692 oz/mol). The level of de-acetylation is preferably from 10 to 99%, with 60 to 99% being especially preferred.

A suitable chitosan is sold, for example, by Kyowa Oil&Fat in Japan under the trade name Flonac^{®}. It has a molecular weight of 300,000 to 700,000 g/mol (10,582 to 24,692 oz/mol) and is de-acetylated to 70 to 80%. A preferred chitosan salt is chitosonium pyrrolidone carboxylate, which, for example, is sold under the name Kytamer^{®} PC by Amerchol in the USA. The chitosan contained therein has a molecular weight of approx. 200,000 to 300,000 g/mol (7,054 to 10,582 oz/mol) and is de-acetylated to 70 to 85%. Quaternated, alkylated, or hydroxyalkylated derivatives, for example, hydroxyethyl or hydroxybutyl chitosans can be considered chitosan derivatives. The chitosans or chitosan derivatives are preferably present in their neutralized or partially neutralized form. The level of neutralization for the chitosan or the chitosan derivative is preferably at least 50%, with 70 to 100% being especially preferred, based on the number of free base groups. In principle, all cosmetically compatible inorganic or organic acids can be used as neutralizers such as formic acid, maleic acid, succinic acid, tartaric acid, citric acid, malonic acid, oxalic acid, and pyrrolidone carboxylic acid, of which the citric acid is preferred.

### Cationic Modified Protein Derivatives and Hydrolysates

Other suitable cationic polymers that can be contained in the permanent shaping composition according to the present invention are cationic modified protein derivatives or cationic modified protein hydrolysates and are, for example, known under the INCl designations Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, or Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, and Hydroxypropyltrimonium Hydrolyzed Vegetable Protein.

Suitable cationically derived protein hydrolysates are substance mixtures, which, for example, receive glycidyl trialkyl ammonium salts or 3-halo-2-hydroxypropyl trialkyl ammonium salts via the conversion of alkaline, acidic, or enzyme-hydrolyzed proteins. Proteins that are used as starting materials for the protein hydrolysates can be of plant or animal origin. Customary starting materials are, for example, keratin, collagen, elastin, soy protein, rice protein, milk protein, wheat protein, silk protein, or almond protein. The hydrolysis results in material mixtures with mole masses in the range of approx. 100 to approx. 50,000. Customary, mean mole masses are in the range of about 500 to about 1,000. It is advantageous if the cationically derived protein hydrolysates have one or two long C8 to C22 alkyl chains and two or one short C1 to C4 alkyl chain accordingly. Compounds containing one long alkyl chain are preferred.

### Cationic Silicone Polymers

Suitable cationic silicon polymers either have at least one least one ammonium group, examples are POLYSILICONE-9; preferred are diquaternary polysiloxanes as defined in the EP 0714654 A1 of the applicant and those having the chemical name dimethylsiloxane, 3-(3-((3-cocoamidopropyl)dimethylammonio)-2-hydroxy-prpoxy)propyl groupterminated acetate (CAS 134737-05-6), defined in the CTFA as QUATERNIUM-80 and sold under the trade names Abil Quat^{®} 3270, Abil^{®} Quat 3272 and Abil^{®} Quat 3474 by the company Th. Goldschmidt AG, Germany; another preferred cationic silicon polymer is aminoethylaminopropylsiloxane/dimethylsiloxan-copolymer emulsion, sold e. g. as GE Toshiba Silicone^{®} and Dow Corning 2-8566^{®} (CTFA: AMODIMETHICONE), another preferred cationic silicon polymer is the polyethylene glycol derivative of aminoethylaminopropylsiloxane/dimethylsiloxan-copolymer (CTFA: PEG-7 AMODIMETHICONE). Also preferred are the following silicone polymers (as defined in CTFA 10^{th} Edition): SILICONE QUATERNIUM-1, SILICONE QUATERNIUM-2, SILICONE QUATERNIUM-2 PANTHENOL, SILICONE QUATERNIUM-3, SILICONE QUATERNIUM-4, SILICONE QUATERNIUM-5, SILICONE QUATERNIUM-6, SILICONE QUATERNIUM-7, SILICONE QUATERNIUM-8, SILICONE QUATERNIUM-9, SILICONE QUATERNIUM-10, SILICONE QUATERNIUM-11 and SILICONE QUATERNIUM-12.

Further silicones for use herein which are preferred are amino functional siloxanes which conform to the general formula (III) 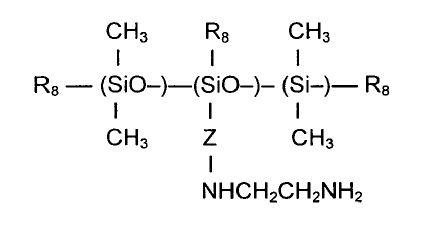 wherein R₈ = OH or CH₃ and Z represents the propyl, isopropyl or isobutyl group. These silicones, e. g., copolymer of aminoethyl aminopropyl siloxane and dimethyl siloxane are available from Dow Corning and sold under the trade name Dow Corning 2-8566 Amino Fluid^{(R)} or as a mixture with polyethylenglycol ether of tridecyl alcohol and cetyl trimethyl ammoniumchloride, sold as Dow Corning 929 Cationic Emulsion^{(R)}.

The most preferred cationic polymers are: The copolymer of hydroxyethyl cellulose and diallyldimethylammonium chloride (POLYQUATERNIUM-4); the copolymer of acrylamide and diallyldimethylammonium chloride (POLYQUATERNIUM-7); vinyl pyrrolidone/dimethylamino ethylmethacrylate methosulfate copolymer (POLYQUATERNIUM-11); the copolymer of acrylamide and betamethacrylyloxyethyl trimethyl ammonium chloride (POLYQUATERNIUM-15); the copolymer of polyvinyl pyrrolidone and imidazolimine methochloride (POLYQUATERNIUM-16); the copolymer of diallyldimethyl ammonium chloride and acrylic acid (POLYQUATERNIUM-22); the copolymer of vinyl pyrrolidone and methacrylamidopropyl trimethyl ammonium chloride (POLYQUATERNIUM-28); the methosulfate of the copolymer of methacryloyloxyethyl trimethylammonium and methacryloyloxyethyl dimethylacetylammonium (POLYQUATERNIUM-35); quaternized hydroxyethyl cellulose (CTFA: POLYQUATERNIUM-10); dimethylsiloxane, 3-(3-((3-cocoamidopropyl)dimethylammonio)-2-hydroxy-prpoxy)propyl groupterminated acetate (CTFA: QUATERNIUM-80); the copolymer of aminoethylaminopropylsiloxane and dimethylsiloxan (CTFA: AMODIMETHICONE); the polyethylene glycol derivative of aminoethylaminopropylsiloxane/dimethylsiloxan-copolymer (CTFA: PEG-7 AMODIMETHICONE); SILICONE QUATERNIUM-7 and SILICONE QUATERNIUM-8.

The preferred quantity for use of the cationic polymers is from about 0.05 to about 15 % by weight, more preferably from about 0.1 to about 8 % by weight and most preferred from about 0.2 to about 3 % by weight of the composition.

### Hair Swelling and Penetration Enhancing Substances

So-called swelling agents and penetrating substances may be added to the hair shaping composition, examples being urea, melamine, ethers, e. g. dipropyleneglycol monomethyl ether; 2-pyrrolidon, imidazolidin-2-one, 1-methyl-2-pyrrolidone; alkali or ammonium thiocyanate, polyvalent alcohols, isopropanol in a quantity of from about 1 to about 30 % by weight.

### Disulfides

It is advantageous if the permanent shaping agent, to avoid making the hair too kinky, contains the disulfide of a hair keratin-reducing compound (thiol), particularly dithioglycolic acid, 2,2'-dithio-bis[N-(3-hydroxypropyl)-acetamid], 2,2'-dithio-bis[N-(propyl)-acetamid], 2,2'-dithio-bis[N-(2-hydroxypropyl)-acetamid], dithiolactic acid and their salts. The preferred quantity for use is from about 0.1 to about 30 % by weight, more preferably from about 0.5 to about 20 % by weight and most preferred from about 1 to about 10 % by weight of the composition, wherein the ratio between the hair keratin-reducing agents and the disulfides is preferably from about 20 : 1 to about 1 : 2, and particularly 10 : 1 to 1 : 1.

### Additives

It is understood that the waving composition may contain all the usual and known additives for such compositions, such as thickeners, such as kaolin, bentonite, fatty acids, higher fatty alcohols, starches, cellulose derivatives, alginates, polyacrylic acid and its derivatives, cellulose derivatives, alginates, vaseline, and paraffin oils; wetting agents or emulsifiers from the classes of anionic, cationic, amphoteric or nonionic surface-active substances, such as fatty alcohol sulfates, fatty alcohol ether sulfates, alkyl sulfonates, alkylbenzene sulfates, quaternary ammonium salts, alkyl betaines, ethoxylated alkylphenols, fatty acid alkanolamides or ethoxylated fatty acid esters; and opacifiers, such as polyethyleneglycol esters; alcohols, such as ethanol, propanol, isopropanol; water-soluble polyhydric alcohols having two or more hydroxyl groups in the molecule. Typical examples of such polyhydric alcohols are dihydric alcohols such as ethylene glycol, 1,2- or 1,3-propanediol, 1,2-pentanediol and glycerine; further, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc. ; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerin monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerin ether, POP glycerin ether, POP glycerin ether phosphoric acid, POP POE pentanerythritol ether; sugars such as D-glucose; solubilizers, stabilizers, buffer substances, perfume oils, dyes, and hair-conditioning and hair-care ingredients such as lanolin derivatives, cholesterol, pantothenic acid, and betaine.

### Cationic Surfactants

Cationic surfactants that can be preferably used in the cosmetic composition of the present invention contain amino or quaternary ammonium moieties. Among the quaternary ammonium-containing cationic surfactant materials useful herein are those of the general formula (IV)

[NR4,R5,R6,R7]⁺ · X⁻

wherein R4 to R7 are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X⁻ is a salt-forming anion such as those selected from halogen, (e. g. chloride, bromide, iodide), acetate, citrate, lactate, glycolate, phosphate nitrate, sulfate, and alkylsulfate radicals.

The aliphatic groups may contain, in addition to carbon and hydrogen atoms, either linkages, and other groups such as amino groups. The longer chain aliphatic groups, e. g. those of about 12 carbons, or higher, can be saturated or unsaturated. Especially preferred are di-long chain (e. g. di C12-C22, preferably C16-C18, aliphatic, preferably alkyl) and di-short chain (e. g. C1 - C3 alkyl, preferably C1 - C2 alkyl) ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydorxy ethyl stearylamine, and arachidylbehenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Preferred cationic surfactants are cetyl trimethyl ammonium salts, as for example Genamin^{®} CTAC, i. e. cetyl trimethyl ammonium chloride, behenyl trimethyl ammonium salts, e. g. behenyl trimethyl ammonium chloride; dimethyl ditallow ammonium salts; stearyl amidopropyl dimethylamine; esterquats as for example tetradecyl betainester chloride, diesterquats as for example dipalmitylethyl dimethylammoniumchloride (Armocare^{®} VGH70 of Akzo, Germany), or a mixture of distearoylethyl hydroxyethylmonium methosulfate and Cetearyl Alkohol (Dehyquart^{®} F-75 of Henkel, Germany).

### Silicone Conditioning Agents

The cosmetic compositions hereof can also include volatile or nonvolatile, soluble or insoluble silicones as conditioning agents. By soluble what is meant is that the silicone conditioning agent is miscible with the aqueous carrier of the composition so as to form part of the same phase. By insoluble what is meant is that the silicone forms a separate, discontinuous phase from the aqueous carrier, such as in the form of an emulsion or a suspension of droplets of the silicone.

Soluble silicones include silicone copolyols, such as dimethicone copolyols, e.g. polyether siloxane-modified polymers, such as polypropylene oxide, polyethylene oxide modified polydimethylsiloxane, wherein the level of ethylene and/or propylene oxide sufficient to allow solubility in the composition.

Preferred, however, are insoluble silicones. The insoluble silicone hair conditioning agent for use herein will preferably have viscosity of from about 1,000 to about 2,000,000 mPa · s at 25°C, more preferably from about 10,000 to about 1,800,000, even more preferably from about 100,000 to about 1,500,000 mPa · s at 25°C. The viscosity can be measured by means of a glass capillary viscometer as set forth in Dow Corning Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof. Specific examples thereof include hexamethyldisiloxane, octamethyltrisiloxane, decamethyltetrasiloxane and hexadecamethylheptasiloxane.

Other insoluble, nonvolatile silicone fluids having hair conditioning properties can also be used. The term "nonvolatile" as used herein shall mean that the silicone has a boiling point of at least about 260°C, preferably at least about 275°C, more preferably at least about 300°C. Such materials exhibit very low or no significant vapor pressure at ambient conditions. The term "silicone fluid" shall mean flowable silicone materials having a viscosity of less than 1,000,000 mPa · s at 25°C. Generally, the viscosity of the fluid will be between about 5 and 1,000,000 mPa · s at 25°C, preferably between about 10 and about 300,000 mPa · s at 25°C.

The preferred silicones are polydimethyl siloxane, polydiethylsiloxane, and polymethylphenylsiloxane. Polydimethylsiloxane is especially preferred. The nonvolatile polyalkylsiloxane fluids that may be used include, for example, polydimethyl siloxanes. These siloxanes are available, for example, from the General Electric Company in their ViscasilR and SF 96 series, and from Dow Corning in their Dow Corning 200^{®} series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

Suitable insoluble, volatile silicone fluids include low molecular weight oligomeric polydimethylsiloxane or cyclic polydimethylsiloxane, having a viscosity of no more than 10 mPa · s at 25°C and a boiling point under atmospheric pressure of no more than 250°C. Volatility can be achieved in linear organopolysiloxanes by selection of oligomeric organopolysiloxanes with at most 6 to 10 silicone atoms in the organopolysiloxanes backbone, e. g. Dow Corning DC200 Fluid, having a viscosity of from about 0.65 to about 2 mPa · s at 25°C. Preferably cylclic organopolysiloxanes having from 3 to 6 silicon atoms are utilized, for example, hexamethylcyclotrisiloxane, octamethylcyclotetrasiloxane, decamethylcyclopentasiloxane, dodecamethylcyclohexasiloxane (e. g. DC 244, DC 245, DC 345 of Dow Corning).

Especially preferred, for enhancing the shine characteristics of hair, are highly arylated silicones, such as highly phenylated polyethyl silicone having refractive indices of about 1.46 or higher, especially about 1.52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e. g. Dow Corning DC-1248^{®}) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 mPa · s. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof. Preferably the silicone hair treating agent comprises a mixture of a polydimethylsiloxane gum, having a viscosity greater than about 1,000,000 mPa · s and polydimethylsiloxane fluid having a viscosity of from about 10 mPa · s to about 100,000 mPa · s at 25°C, wherein the ratio of gum to fluid is from about 30:70 to about 70:30, preferably from about 40:60 to about 60:40.

Silicone conditioning polymers that are specially preferred are CTFA: DIMETHICONE BISAMINO HYDROXYPROPYL COPOLYOL, BISAMINO PEG/PPG-41/3 AMINOETHYL PG-PROPYL DIMETHICONE, dihydroxypolydimethylsiloxane (CTFA: DIMETHICONOL). Also preferred are volatile silicones such as e. g. CTFA: DIMETHICONE, DIMETHICONE COPOLYOL and CYCLOMETHICONE.

The silicone hair conditioning agent can be used in the compositions hereof at levels of from about 0.1 % to about 20% by weight of the composition, preferably from about 0.5 % to about 15%, more preferably from about 1% to about 10% and most preferably from about2% to about 8% by weight.

### Additional Conditioning Agents

The cosmetic compositions of the present invention can also comprise one or more additional conditioning agents, such as those selected from the group consisting of liquid oils and fats such as avocado oil, tsubaki oil, turtle oil, Macademia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, palm oil, tallow, sheep fat, hardened tallow, palm kernel oil, jojoba oil, lard, ox bone fat, wood wax kernel oil, hardened castor oil; waxes such as beeswax, apple wax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons, nonvolatile hydrocarbons and hydrocarbon esters such as fluid paraffin, solid paraffin, vaseline, ozocerite, squalane, pristan, ceresin, squalane, petrolatum, isododecane, microcrystalline wax; fatty acid oils, ester oils such as cetyl octanoate, isopropyl myristate; betaine, carnitin, carnitin esters, creatine, amino acids, peptides, proteines, vitamines, phospholipides, e. g. lecithines or ceramides. Useful are also quaternary benzyl salts such as lauryl-dimethyl-ethylbenzyl-ammonium chloride (QUATERNIUM-14); imidazolidinyl derivatives as for example CTFA: QUATERNIUM-87 (Rewoquat^{®} W 575 of Witco, Germany); N-(3-chloroallyl)-hexaminium chloride (QUATERNIUM-15); a quaternary ammonium compound sold under the trade name Finquat CT by the company Finetex (CTFA: QUATERNIUM-75; see CTFA Tenth Edition, page 1604).

The amount of the above conditioning agents preferably ranges from about 0.1 to 30% by weight, better still from about 0.5 to 15% by weight and most preferred from about 1 to 10% by weight of the total weight of the cosmetic composition. These ranges include all specific values and sub-ranges there between, including 3, 8, 10, 15, 20, 25 and 30% by weight.

### Fatty Alcohols

The cosmetic compositions of the present invention may also comprise a nonvolatile low melting point fatty alcohol. The fatty alcohols hereof have a melting point of 30°C or less, preferably about 25°C or less, more preferably about 22°C or less. The unsaturated fatty alcohols hereof are also nonvolatile. By nonvolatile what is meant is they have a boiling point at 1..0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, preferably less than about 5% by total weight of the unsaturated straight chain fatty alcohol more preferably less than about 2%, most preferably less than about 1%. Preferably, the unsaturated straight chain fatty alcohols will have an aliphatic chain size of from C12-C22, more preferably from C12-C18, most preferably from C16-C18. Exemplary alcohols of this type include oleyl alcohol, and palmitoleic alcohol. The branched chain alcohols will typically have aliphatic chain sizes of from C12-C22, preferably C14-C20, more preferably C16-C18.

Exemplary branched chain alcohols for use herein include stearyl alcohol, cetyl alcohol, isostearyl alcohol, octyl dodecanol, and octyl decanol.

Examples of saturated C8-C12 straight chain alcohols include octyl alcohol, caprylic alcohol, decyl alcohol, and lauryl alcohol. The low melting point fatty alcohols hereof are used at a level of from about 0.1 % to about 15%, by weight of the composition, more preferably from about 0.2% to about 11 %, most preferably from about 0.5% to about 3%.

It may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10.

The total amount of fatty alcohols in the composition is preferably about 0.5 to about 15.0 % by weight, more preferably from about 1.0 to about 12.0 % by weight, and most preferably from about 1.5 to about 11.0 % by weight.

### Other Ingredients

The cosmetic compositions herein can contain a variety of other optional components suitable for rendering such compositions more cosmetically or aesthetically acceptable or to provide them with additional usage benefits. Such conventional optional ingredients are well-known to those skilled in the art. The composition of the invention may thus comprise lipophilic or hydrophilic adjuvants which are standard in the cosmetics or dermatological fields, such as surfactants, in particular foaming surfactants, preservatives, antioxidants, sequestering agents, solvents, fragrances, fillers, screening agents, odor absorbers, coloring materials and lipid vesicles.

A wide variety of additional ingredients can be formulated into the present cosmetic composition. These include: hair-hold polymers, detersive surfactants such as anionic, nonionic, amphoteric, and zwitterionic surfactants; additional thickening agents and suspending agents, such as xanthan gum, guar gum, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; UV-filters and sunscreens, e. g. such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; agents for combating free radicals; preservatives such as benzyl alcohol, methyl paraben, propyl paraben and imidazolidinyl urea; polyvinyl alcohol; pH adjusting agents, such as citric acid, formic acid, glyoxylic acid, acetic acid, lactic acid, pyruvic acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes, sequestering agents, such as disodium ethylenediamine tetra-acetate, and polymer plasticizing agents, such as glycerin, disobutyl adipate, butyl stearate, and propylene glycol.

From the nonionic surfactants optionally used those are preferred having an HLB (Hydrophilic Lipophilic Balance) of greater than 12 and the primary emulsion comprises at least one nonionic surfactant having an HLB of less than 8. The nonionic surfactant of HLB>12 optionally present in the emulsion may be, for example, an ethoxylated or ethoxylated/propoxylated fatty alcohol with a fatty chain comprising from 12 to 22 carbon atoms, ethoxylated sterols, such as stearyl- or lauryl alcohol (EO-7); PEG-16 soya sterol or PEG-10 soya sterol, polyoxyethylene polyoxypropylene block polymers (poloxamers) and their mixtures. Ethoxylated sterols and of poloxamers are preferred. The nonionic surfactant of HLB<8 can be chosen in particular from glyceryl esters, such as mono-, di- or triglyceryl mono-, di- or tri-isostearate or -oleate, sugar esters, such as sucrose or methyl glucose mono- or diisostearate or -oleate, alkylpolyglucoside ethers, such as sorbitan isostearate, oleyl- or isostearylpolyglucoside; polyoxyethylene (20) sorbitan monostearate (CTFA: Polysorbat-60), and their mixtures. Sugar esters and alkylpolyglucoside ethers are preferred.

The amount of nonionic surfactant in the emulsion and the cosmetic composition preferably can range from about 0.1 % to about 5% by weight and more preferably from about 1% to about 3% by weight of the total weight of the emulsion.

The additives are used in quantities usual for such purposes; for example, the wetting agents and emulsifiers in concentrations of a total of 0.2% to 30 % by weight, the alcohols in a total quantity of 0.1 % to 20 % by weight, the opacifiers, perfume oils and dyes in a quantity of 0.01 % to 1 % by weight each, the buffer substances in a total quantity of 0.1 % to 10 % by weight, and sugars, solubilizers, stabilizers and hair-conditioning and hair-care ingredients in a quantity of 0.1 % to 5% by weight each, while the thickeners and solubilizers may be contained in this composition in a total quantity of 0.5 to 20 % by weight. Surfactants are preferably contained at levels of from about 0.1 % to about 5%, more preferably from about 0.2% to about 1,5%, most preferably from about 0.4% to about 0.8%, by weight of the composition.

### pH-Value

By varying the pH value, a composition can be made available that is universally suitable for every hair structure, optionally with the additional application of heat. The composition brings about an elastic, durable and uniform waving from the root of the hair to the ends, without eliciting allergic or sensitizing reactions.

The hair shaping composition preferably has a pH value of from 2.0 to 9.5 and especially preferably from 5.5 to 9.0. As an alkalizing agent or agent for adjusting the pH value, ammonia or caustic soda is especially suitable, but water-soluble, physiologically tolerable salts of organic and inorganic bases can also be considered, such as ammonium hydrogen carbonate, ammonia, monoethanolamine, ammonium carbonate. Further suitable alkalizing agents may be selected from the group consisting of 2-amino-2-methyl-1-propanol, 2-amino-2-methyl-1,3-propanediol, 2-amino-2-ethyl-1,3-propanediol, tris(hydroxylmethyl)-aminomethane, 2-amino-1-butanole, tris-(2-hydroxypropyl)-amine, 2,2-iminobisethanol, lysine, iminourea (guanidine carbonate), tetrahydro-1,4-oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide and magnesium oxide.

To establish an acidic pH value, hydrochloric acid, phosphoric acid, acetic acid or citric acid can be used in particular. The acidic pH can be also adjusted with a buffer such as a phosphate buffer, a TRIS buffer or a citric buffer. The buffers may be used alone or in combination with an acid.

If the shaping composition is adjusted to be acidic (for example to a pH = 5.0 to 6.9), then esters of mercapto carboxylic acids may be present as co-agents such as, for example, monothioglycol acid glycol esters or -glycerin esters, with mercapto acetamides or 2-mercaptopropionic acid amides being preferred, in a concentration of from 1 to 14 percent by weight; or the salts of the sulfuric acid, for example, sodium, ammonium, or monoethanol ammonium sulfite, in a concentration of from 3 to 8 percent by weight (calculated as SO₂).

### Viscosity

The hair shaping composition of the present invention preferably has a viscosity at 25°C of about 0.1 mPa · s to about 10,000 mPa · s, preferably from about 1 mPa · s to about 5,000 mPa · s, more preferably from about 5 mPa · s to about 3,500 mPa · s. Viscosity is determined - if not otherwise defined - by HAAKE Rotation Viscometer VT 550 with cooling/heating vessel and sensor systems according to DIN 53019 (MV-DIN, SV-DIN), shear rate is 12.9 s⁻¹.

### Packages

The shaping composition may be sold in single- or dual- or triple-component packages; the composition may be in the form of an aqueous solution, or an emulsion, or in thickened water-based form, especially a creme, gel, foam or paste.

### Production

The production of the preferred N-(3'-hydroxypropyl)-2-mercaptoacetamide of formula (I) is done by reacting the 3-aminopropanole at a temperature of not above 30° C with methylthioglycolate in a protective gas atmosphere, extraction in a suitable solvent, and ensuing flash distillation.

### Method for Permanent Hair Shaping

The method of permanent shaping of human hair according to the invention is defined in claim 13.

Further details of the method are given as follows: First the hair (which is washed and towel-dried) is separated into multiple sections and then these sections are rolled onto curlers. The curlers used for permanent waves have a diameter of about 5 to 13 mm (0.17 to 0.44 in), while the curlers used for straightening must have a diameter greater than 13 mm (0.44 in). After the rolling on curlers is finished, the curlers are thoroughly wetted down using the required quantity of the permanent shaping composition, preferably 60 to 120 g.

The amount of time the permanent shaping composition stays on the hair is from about 1 to about 30 minutes, preferably from about 5 to about 20 minutes. This action time can be shortened by adding heat via the use of a heat radiator or a hood dryer.

After the action time has elapsed that is sufficient for the permanent shaping, which is depending on hair quality, the pH value, the shaping effectiveness of the color-providing shaping agent, the desired level of change as well as on the application temperature, the hair is rinsed with water or treated with an intermediate treatment agent. Thereafter the hair is oxidatively post-treated ("fixed"). The fixing composition is used in a quantity of about 50 to 200 g (1.76 to 7.05 oz), preferably 80 to 100 g (2.27 to 3.52 oz), depending on hair thickness and length. Any oxidizing agent that has been used before in fixing compositions can be used for the fixation. Examples of such oxidizing agents are potassium bromate, sodium bromate, sodium perborate, dehydroascorbic acid, hydrogen peroxide, and urea peroxide. Hydrogen peroxide is preferred. The concentration of the oxidizing agent varies depending on application time (normally 1 to 40 minutes, or preferably 5 to 20 minutes) and application temperature (25 to 50 degrees Celsius (77 °F to 122 °F)). The concentration of the oxidant varies, depending on the application time (as a rule, 5 to 15 minutes) and the application temperature. Normally, oxidizing agents are used in a concentration of about 0.5 to 12.0 % by weight, preferably 1 to 3 % by weight, in the aqueous fixing composition.

The fixing composition can obviously contain other materials, for example, weak acids or peroxide stabilizers. The fixing composition can naturally also include other substances, such as wetting agents, hair-care substances such as cationic polymers, weak acids, buffer substances or peroxide stabilizers, and may be in the form of an aqueous solution, an emulsion, or a thickened water-based form, in particular a cream, gel or paste. These typical additives may be contained in the fixing composition in a quantity of 0.1 to 10 % by weight, in particular.

Both the permanent shaping composition of the invention and the fixing composition can be present in the form of an aqueous solution or an emulsion, as well as in a thickened form on an aqueous basis, particularly as a cream, gel, or paste. It is also possible to fill these compositions into aerosol cans under pressure and to release them as aerosol foam. It is especially preferred that the fixing composition be in low viscosity liquid form. It is preferred that the fixing composition be an oxidation agent-containing, liquid preparation with a viscosity of from 1 to 1,000 mPa · s at 25 degrees Celsius (77 °F), wherein viscosity of from 2 to 500 mPa · s at 25 degrees Celsius (77 °F) is especially preferred. The viscosity values are based on measurements with a Haake rotational viscometer, type VT 550, at a shear speed of 12.9 per second.

After an action period required for the fixing agent of from about 3 to about 15 minutes, preferably 5 to 10 minutes, the curlers are removed. If necessary, the hair after being taken down from the curlers is oxidatively post-treated yet again, than the fixing agent is rinsed from the hair with water. The hair is optionally treated with a known acidic rinse. It is advantageous if the hair is then finally shaped into a water wave and then dried.

It is completely surprising that the method and composition for permanent hair shaping of the invention leads to improved results in a number of respects compared to the compositions comrising the widely used waving agent thioglycolic acid and its salts with at least one cationic polymer, but mainly in that the uniformity of curls of the hair treated is significantly better over the entire length of the hair.

The composition and method of the invention does not have the disadvantages of glyceryl thioglycolate and thioglycolic acid and its salts but possesses both advantages of displaying no sensitization problem and superior hair care effects. The excellent condition of hair treated with composition and method of the invention was shown by an improved soft feel of wet and dry hair, an improved uniformity of the curls from the roots to the tips, an improved elasticity of hair and durable waving of the hair.

The following examples are intended to explain the subject of the invention in further detail, but without limiting the subject to these examples.

### Examples

Example 1 Low Alkaline Permanent Waving Composition for Normal Hair

| | |
|---|---|
| 12.00 g | N-(3'-hydroxypropyl)-2-mercaptoacetamide |
| 0.20 g | ammonia (25 % aqueous solution) |
| 3.50 g | ammonium hydrogen carbonate |
| 0.10 g | Amerchol Polymer SL^{®}-60 (CTFA: POLYQUATERNIUM-67) |
| 0.20 g | vinylpyrrolidon/dimethylaminoethylmethacrylat methosulfate |
| | copolymer (CTFA: POLYQUATERNIUM-11) |
| 1.00 g | 1,3-butandiol |
| 1.50 g | 1,2-propylenglykol |
| 2.00 g | dipropylenglykol monoethylether |
| 3.50 g | urea |
| 1.00 g | castor oil, ethoxylated with 35 moles ethylene oxide |
| | Chremophor^{®} EL (CTFA: PEG-35 CASTOR OIL) |
| 1.00 g | coconut alcohol, ethoxylated with 10 moles ethylene oxide |
| | Genapol^{®} C 100 (CTFA: COCETH-10) |
| ad 100.00 g | water |

The pH of the composition is adjusted with ammonia to 8.4.

After winding the hair on curlers the above permanent waving agent is uniformly applied onto the curlers. The permanent waving agent is left on the curlers for 10 minutes. An infrared drying hood is used at a temperature of 40°C. During the treating time a much better and more pleasant smell is noticed by the hairdresser as well as by the customer compared to permanent waving agents commonly used. The hair is than rinsed with lukewarm water. Thereafter 80g of a fixing agent of the formula given below is applied onto the curler.

### Fixing Agent

| | |
|---|---|
| 4.00 g | hydrogen peroxide, 50% aqueous solution |
| 0.10 g | salicylic acid |
| 0.20 g | dinatrium hydrogen phosphate |
| 0.15 g | o-phosphoric acid |
| 1.00 g | castor oil ethoxylated wit 35 moles of ethylene oxide |
| 0.10 g | vinylpyrrolidon/styrene-copolymer |
| 0.10 g | perfume oil |
| ad 100.00 g | water |

The fixing agent is left on the hair for 8 minutes. Finally the hair is again rinsed with lukewarm water and the curlers are removed. The hair shows in dry and wet condition a well groomed look, a good touch and a fine wet combing property. It displays a good elasticity from the tips to the roots. Even after 3 days the hair keeps its pleasant smell. During or after the hair treating procedure described above neither irritations of the skin nor other effects on the skin were realized.

### Example 2 Low Alkaline Permanent Waving Composition for Colored Hair

| | |
|---|---|
| 5.50 g | N-(3'-hydroxypropyl)-2-mercaptoacetamide |
| 0.50 g | 2,2'-dithio-bis[(N-(3-hydroxypropyl)-acetamide] |
| 0.20 g | ammonia, 25 % aqueous solution |
| 2.60 g | ammonium hydrogen carbonate |
| 0.20 g | Dimethylsiloxane, 3-(3-((3-cocoamidopropyl)dimethylammonio)-2-hydroxyprpoxy)propyl groupterminated acetate; Abil Quat^{®} 3272 (CTFA:QUATERNIUM-80; CAS 134737-05-6) |
| 1.00 g | aminofunctional siliconpolymer (CTFA: METHOXY PEG/PPG-7/3 AMINOPROPYL DIMETHICONE) |
| 0.30 g | styrene/vinylpyrrolidon-copolymer |
| | (CTFA: STYRENE/VP COPOLYMER) |
| 1.50 g | 1,2-propylenglykol |
| 2.00 g | dipropyleneglykol monoethyl ether |
| 2.00 g | urea |
| 1.00 g | hydrogenated castor oil, ethoxylated wit 40 moles of ethylene oxide (CTFA: PEG-40 HYDROGENATED CASTOR OIL) |
| 1.00 g | Genapol^{®} C 100 (CTFA: CocETH-10) |
| 0.50 g | perfume oil |
| ad 100.00 g | water |

The pH of the composition is adjusted with ammonia to 8.2.

After winding the hair on curlers the above permanent waving agent is uniformly applied onto the curlers. The permanent waving agent is left on the curlers for 12 minutes. An infrared drying hood is used at a temperature of 40°C. During the treating time a much better and more pleasant smell is noticed by the hairdresser as well as by the customer compared to permanent waving agents commonly used. The hair is than rinsed with lukewarm water. Thereafter 80g of a fixing agent of example 1 is applied onto the curler.

The fixing agent is left on the hair for 6 minutes. Finally the hair is again rinsed with lukewarm water and the curlers are removed. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Example 3 Low Alkaline Permanent Waving Composition for Colored Hair

| | |
|---|---|
| 7.20 g | N-(3'-hydroxypropyl)-2-mercaptoacetamide |
| 1.00 g | 2,2'-dithio-bis[(N-(3-hydroxypropyl)-acetamide] |
| 0.20 g | ammonia (25 % aqueous solution) |
| 2.60 g | ammonium hydrogen carbonate |
| 1.50 g | aminoethylaminopropylsiloxane/dimethylsiloxan-copolymer emulsion; Dow Corning 2-8566^{®} |
| | (CTFA: AMODIMETHICONE) |
| 0.20 g | cetyltrimethylammoniumchloride; |
| | Genamin^{®} CTAC (CTFA: CETRIMONIUM CHLORIDE) |
| 1.50 g | 1,2-propylene glycol |
| 2.00 g | dipropyleneglycol monoethylether |
| 1.00 g | castor oil ethoxylated with 35 mol ethylenoxide (CTFA: PEG-35 CASTOR OIL) |
| 1.00 g | Genapol^{®} C 100 (CTFA: COCETH-10) |
| 0.30 g | Antara^{®} 430 (CTFA: Styrene/VP Copolymer) |
| 0.50 g | perfume oil |
| ad 100.00 g | water |

The pH of the composition is adjusted with ammonia to 8.2.

After winding the hair on curlers the above permanent waving agent is uniformly applied onto the curlers. The permanent waving agent is left on the curlers for 8 minutes. An infrared drying hood is used at a temperature of 38°C. During the treating time a much better and more pleasant smell is notices by the hairdresser as well as by the customer compared to commonly used permanent waving agents.
The hair is than rinsed with lukewarm water. Thereafter 80g of a fixing agent of example 1 is applied onto the curler.

The fixing agent is left on the hair for 7 minutes. Finally the hair is again rinsed with lukewarm water and the curlers are removed. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

The composition for permanent shaping of hair of the invention causes less hair damage than compositions containing thioglycolic acid and cationic polymer. This was shown by breaking force measurements.

### Example 4 Low Alkaline Permanent Waving Composition for Unmanageable Hair

| | |
|---|---|
| 10.08 g | N-(3'-hydroxypropyl)-2-mercaptoacetamide |
| 0.50 g | 2,2'-dithio-bis[(N-(3-hydroxypropyl)-acetamide] |
| 6.00 g | ammonia (25 % aqueous solution) |
| 3.50 g | ammonium hydrogen carbonate |
| 0.20 g | cetyltrimethylammonium chloride |
| 0.10 g | Abil Quat^{®} 3270 (CTFA: QUATERNIUM-80) |
| 0.30 g | quaternized vinylpyrrolidon/dimethylaminoethylmethacrylate-copolymer (POLYQUATERNIUM-11) |
| 1.00 g | 1,3-butandiol |
| 1.50 g | 1,2-propylenglycol |
| 2.00 g | dipropylenglycol monoethylether |
| 3.50 g | urea |
| 1.50 g | oleic alcohol, ethoxylated with 10 mol of ethylenoxide |
| | (CTFA: OLETH-10) |
| 1.00 g | Genapol^{®} C 100 (CTFA: CocETH-10) |
| 0.60 g | perfume oil |
| ad 100.00 g | water |

The pH of the composition is adjusted with ammonia to 8.9.

After winding the hair on curlers the above permanent waving agent is uniformly applied onto the curlers. The permanent waving agent is left on the curlers for 14 minutes. An infrared drying hood is used at a temperature of 42°C. During the treating time a much better and more pleasant smell is notices by the hairdresser as well as by the customer compared to commonly used permanent waving agents. The hair is than rinsed with lukewarm water. Thereafter 80g of a fixing agent of example 1 is applied onto the curler.

The fixing agent is left on the hair for 7 minutes. Than the curlers are removed and the hair is post neutralized for 5 min with 40g of the fixing agent of example 1. Finally the hair is again rinsed with lukewarm water. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Example 5 2-Component Drop Free Permanent Waving Composition for Colored Hair

### Component 1

| | |
|---|---|
| 0.10 g | ammonia (25 % aqueous solution) |
| 2.50 g | ammonium hydrogen carbonate |
| 1.20 g | aminoethylaminopropylsiloxane/dimethylsiloxan-copolymer emulsion; Dow Corning 2-8566^{®} (CTFA: AMODIMETHICONE) |
| 0.20 g | (CTFA: LAURDIMONIUM HYDROXYPROPYL HYDROLYZED WHEAT PROTEIN) |
| 0.10 g | cetyltrimethylammonium chloride |
| | (CTFA: CETRIMONIUM CHLORIDE) |
| 0.40 g | methylvinylimidazoliumchlorid/vinylpyrrolidon copolymer |
| | (CTFA: POLYQUATERNIUM-16) |
| 1.50 g | 1,2-propylenglykol |
| 3.00 g | urea |
| 1.80 g | oleic alcohol, ethoxylated with 20 mol of ethylenoxide |
| | (CTFA: OLETH-20) |
| 0.40 g | perfume oil |
| ad 100.00 g | water |

### Component 2

| | |
|---|---|
| 93.00 g | N-(3'-hydroxypropyl)-2-mercaptoacetamide (90%aqueous solution) |
| 7.00 g | 2,2'-dithio-bis[(N-(3-hydroxypropyl)-acetamide] |

Immediately before use 60 g of Component 1 are mixed with 15 g of Component 2. The pH-value of the ready to use hair shaping composition is 7.3. 75 g of the hair shaping composition are uniformly applied on the hair wound on curlers of 8 mm diameter. The permanent waving agent is left on the curlers for 20 minutes at room temperature (25°C). The hair is than rinsed with lukewarm water. Thereafter 80g of a fixing agent of example 1 is applied onto the curler. The fixing agent is left on the hair for 8 minutes. Thereafter the hair is again rinsed with lukewarm water and the curlers are removed.

The hair shows a well groomed look, a good touch, fine wet combing properties and a uniform curl all over the head.

### Example 6 Permanent Waving Composition for Curling the Hair Regrowth

| | |
|---|---|
| 12.60 g | N-(3'-hydroxypropyl)-2-mercaptoacetamide |
| 4.00 g | ammonia (25 % aqueous solution) |
| 5.00 g | ammonium hydrogen carbonate |
| 4.00 g | cetylstearyl alcohol (Lanette^{®} O) |
| 1.00 g | Cremophor^{®} A 25 |
| 0.50 g | lauryl-dimethyl-ethylbenzyl-ammonium chloride |
| | (QUATERNIUM-14) |
| 0.10 g | Amerchol Polymer SL^{®}-60 (CTFA: POLYQUATERNIUM-67) |
| 1.00 g | Dimethylsiloxane, 3-(3-((3-cocoamidopropyl)dimethylammonio)-2-hydroxyprpoxy)propyl groupterminated acetate; |
| | Abil Quat^{®} 3474 (CTFA: QUATERNIUM-80; CAS 134737-05-6) |
| 0.50 g | perfume oil |
| ad 100.00 g | water |

The pH of the gel composition is adjusted to 8.6 with ammonia.

After winding the hair on curlers, the above permanent waving agent in form of a gel is uniformly applied with a brush on the re-growth (upper layer) of curlers. The permanent waving agent is left on the curlers for 10 minutes at 25°C. During the treating time a much better and more pleasant smell is notices by the hairdresser as well as by the customer compared to commonly used permanent waving agents. The hair is than rinsed with lukewarm water. Thereafter 60g of a fixing agent of example 1 is applied onto the curler.

The fixing agent is left on the hair for 10 minutes. Finally the hair is again rinsed with lukewarm water and the curlers are removed. The hair shows a good wave in the re-growth area, a good touch and excellent elasticity.

Example 7 2-Phase-Permanent Waving Composition for Equalizing Large Structural Differences on the Hair

### Phase 1

| | |
|---|---|
| 7.20 g | N-(3'-hydroxypropyl)-2-mercaptoacetamide |
| 0.20 g | ammonia (25 % aqueous solution) |
| 2.60 g | ammonium hydrogen carbonate |
| 1.50 g | Silsoft^{®} A-553 (CTFA: DIMETHICONE BISAMINO |
| | HYDROXYPROPYL COPOLYOL) |
| 0.20 g | cetyltrimethyl ammonium chloride |
| 1.50 g | 1,2-propylenglycol |
| 2.00 g | dipropylenglycol monoethylether |
| 1.00 g | Chremophor^{®} EL (CTFA: PEG-35 CASTOR OIL) |
| 1.00 g | Genapol^{®} C 100 (CTFA: COCETH-10) |
| 0.30 g | Antara 430 (CTFA: Styrene/VP Copolymer) |
| 0.50 g | perfume oil |
| ad 100.00 g | water |

The pH of the composition is adjusted to 8.2 with ammonia.

### Phase 2

| | |
|---|---|
| 12.60 g | N-(3'-hydroxypropyl)-2-mercaptoacetamide |
| 4.50 g | ammonia (25 % aqueous solution) |
| 5.00 g | ammonium hydrogen carbonate |
| 4.00 g | Lanette O |
| 1.00 g | Chremophor A 25 |
| 0.50 g | Ultrasil^{®} A-23 (CTFA: PEG-7 AMODIMETHICONE) |
| 1.50 g | Dimethylsiloxane, 3-(3-((3-cocoamidopropyl)dimethyl-ammonio)-2-hydroxyprpoxy)propyl groupterminated acetate; Abil Quat^{®} 3474 (CTFA: QUATERNIUM-80; CAS 134737-05-6) |
| | |
| | |
| 0.50 g | perfume oil |
| ad 100.00 g | water |

The pH of the composition is adjusted to 8.6 with ammonia.

After winding the hair on curlers the above permanent waving liquid (Phase 1) of pH = 8.2, having a viscosity of 7 mPa · sec at 25°C, is uniformly applied onto the curlers. Immediately thereafter on each of the curlers the above permanent waving gel (Phase 2) of pH = 8.6, having a viscosity of 2.900 mPa · s at 25°, is applied with a brush on the hair re-growth. The permanent waving agent is left on the curlers for 10 minutes at room temperature (25°C). During the treating time a much better and more pleasant smell is noticed by the hairdresser as well as by the customer compared to commonly used permanent waving agents. The hair is than rinsed with lukewarm water.

Thereafter 60g of a fixing agent of example 1 is applied onto the curler. The fixing agent is left on the hair for 10 minutes. Finally the hair is again rinsed with lukewarm water and the curlers are removed. The hair exhibits a uniform curl from the tips to the roots and an excellent elasticity and the hair tips show a good structure. The hair has a good touch and fine wet combing properties. During or after the hair treating procedure described above neither irritations of the skin nor other effects on the skin were realized.

### Example 8 Aerosol Permanent Waving Cream for Curling the Hair Regrowth

| | |
|---|---|
| 12.60 g | N-(3'-hydroxypropyl)-2-mercaptoacetamide |
| 0.50 g | 2,2'-dithio-bis[(N-(3-hydroxypropyl)-acetamide] |
| 4.50 g | ammonia (25 % aqueous solution) |
| 5.00 g | ammonium hydrogen carbonate |
| 4.00 g | Lanette^{®}O |
| 1.00 g | Chremophor^{®} A 25 |
| 0.50 g | aminoethylaminopropylsiloxane/dimethylsiloxan-copolymer emulsion; GE Toshiba Silicone (CTFA: AMODIMETHICONE) |
| 0.10 g | Genamin^{®} KDMP (CTFA: BEHENTRIMONIUM CHLORIDE) |
| 1.50 g | Dimethylsiloxane, 3-(3-((3-cocoamidopropyl)dimethylammonio)-2-hydroxyprpoxy)propyl group terminated acetate; |
| | Abil Quat^{®} 3474 (CTFA: QUATERNIUM-80; CAS 134737-05-6) |
| 0.50 g | perfume oil |
| ad 100.00 g | water |

The pH of the composition is adjusted to 8.6 with ammonia.

### Compounding:

All usual propellants may be used, dimethyl ether is preferred. The above cream is blended with the same weight amount (50 : 50) of the propellant dimethyl ether.

### Application Procedure

After winding the hair on curlers, the above permanent waving agent in form of a creme is uniformly applied from an aerosol container through a spray nozzle onto on the re-growth (upper layer) of the curlers. The permanent waving cream is left on the curlers for 12 minutes at 25°C. During the treating time a much better and more pleasant smell is notices by the hairdresser as well as by the customer compared to commonly used permanent waving agents. The hair is than rinsed with lukewarm water. Thereafter 60g of a fixing agent of example 1 is applied onto the curlers. The fixing agent is left on the hair for 10 minutes. Than the hair is again rinsed with lukewarm water and the curlers are removed.

Since only the hair re-growth was curled the hair now exhibits a uniform curl from the tips to the roots and has an excellent elasticity. The hair tips show a good structural condition. The hair has an excellent touch and fine wet combing properties. During or after the hair treating procedure described above neither irritations of the skin nor other effects on the skin were realized. Further the customer benefits from the non-dripping properties of the waving cream.

### Example 9 Permanent Waving Composition for Dyed Hair

| | |
|---|---|
| 10.80 g | N-propyl-2-mercaptoacetamide |
| 2.00 g | 2,2'-dithio-bis[(N-(3-hydroxypropyl)-acetamide] |
| 1.00 g | copolymer of dimethyldiallyl ammonium chloride and acrylic acid; |
| | Merquat^{®} 280 (POLYQUATERNIUM-22); |
| 0.40 g | ammonia, 25% aqueous solution |
| 2.00 g | ammonium hydrogen carbonate |
| 5.00 g | isopropanol |
| 2.50 g | 1,2-propylene glycol |
| 2.50 g | 1,2-pentanediol |
| 1.00 g | isooctylphenol, ethoxylated with 10 mol ethylene oxide |
| 0.30 g | perfume oil |
| 0.10 g | vinylpyrrolidone/styrene copolymer |
| | (Antara.sup.R 430, GAF Corp., New York) |
| ad 100.00 g | water |

The pH of the composition is adjusted to 7.3 with ammonia.

Hair previously damaged by coloring treatments is washed with a shampoo, towel-dried, and set on curlers with a diameter of 8 mm. Next, the hair waving composition described above is applied evenly onto the hair wound on the curlers. The hair is then covered with a plastic hood and heated for 10 minutes under a dryer hood at a temperature of 45° C. The covering is then removed; the hair is rinsed with water and oxidatively post-treated with 100 g of a 3% aqueous hydrogen peroxide solution. After the curlers are removed, the hair is rinsed again with water, set for a water wave, and then dried. The result of this treatment is a uniform, elastic and durable wave of the hair.

### Example 10 Permanent Waving Composition for Normal Hair

| | |
|---|---|
| 14.50 g | N-propyl-2-mercaptoacetamide |
| 0.20 g | 2,2'-dithio-bis[(N-(3-hydroxypropyl)-acetamide] |
| 0.30 g | Merquat 550 L (CTFA: Polyquaternium-22) |
| 0.40 g | Silsoft^{®} A-858; Organosilicone Copolymer |
| | (CTFA: DEA PG-PROPYL PEG/PPG-18/21 DIMETHICONE) |
| 8.90 g | ammonia, 25% aqueous solution |
| 4.00 g | ammonium hydrogen carbonate |
| 5.00 g | 1,2-propylene glycol |
| 1.00 g | 1,3-butanediol |
| 2.00 g | dipropyleneglycol mono ethyl ether |
| 4.00 g | urea |
| 2.50 g | hydrogenated castor oil, ethoxylated with 40 mole ethylene oxide |
| 2.50 g | lauryl alcohol, ethoxylated with 40 mole ethylene oxide |
| | (CTFA: Laureth-4) |
| 0.10 g | vinylpyrrolidone/styrene copolymer |
| | (Antara.sup.R^{®} 430, GAF Corp., New York) |
| 0.50 g | perfume oil |
| ad 100.00 g | Water |

The pH of the composition is adjusted to 8.4 with ammonia.

Normal hair, not previously damaged, is washed, dried with a hand towel, and wound onto curlers with a diameter of 6 mm. After that the hair is moistened thoroughly and evenly with the above-described hair waving composition. After an action time of 15 minutes, the hair is rinsed thoroughly with water and then oxidatively post-treated with 80 grams of a 3% aqueous hydrogen peroxide solution. After the curlers are taken out, the hair is rinsed again with water, set for a water wave, and then dried. The thus-treated hair has a uniform, lively curliness.

### Example 11 Permanent Waving Composition for Normal Hair

| | |
|---|---|
| 5.50 g | N-propyl-2-mercaptoacetamide |
| 4.50 g | N-(2'-hydroxypropyl)-2-mercaptoacetamide |
| 0.30 g | 2,2'-Dithiobis(N-propyl-acetamide) |
| 0.80 g | ammonia, 25% aqueous solution |
| 5.00 g | ammonium hydrogen carbonate |
| 1.00 g | 1,2-propanediol |
| 2.00 g | D-glucose |
| 1.50 g | isooctylphenol, ethoxylated with 10 mole ethylene oxide |
| 0.40 g | Luviquat^{®} FC 370 (CTFA: POLYQUATERNIUM-16) |
| 1.00 g | pentylen glycol (CTFA: PENTYLEN GLYCOL) |
| 0.50 g | perfume oil |
| 0.10 g | Vinylpyrrolidone/styrene mixed polymer |
| | (Antara.sup.R 430^{®}, GAF Corp., New York) |
| ad 100.00 g | water |

The pH of the composition is adjusted to 8.3 with ammonia.

Normal, not previously damaged hair, is washed, dried with a towel, and wound onto curlers with a diameter of 6 mm. Thereafter the hair is moistened thoroughly and evenly with the above-described hair waving composition. After an action time of 20 min, the hair is rinsed thoroughly with water and then oxidatively post-treated with 80 g of a 3% aqueous hydrogen peroxide solution. Than the curlers are removed, the hair is rinsed again with water, set for a water wave, and then dried. The thus-treated hair has a uniform, lively curliness.

### Example 12 Dual-Component Permanent Waving Composition for Normal Hair Component A

| | |
|---|---|
| 2.00 g | diethyleneglycol monoethyl ether |
| 2.00 g | 1-Methoxypropanol |
| 1.50 g | 1,2-propylene glycol |
| 0.50 g | oleyl alcohol polyethyleneglycol ether - 5EO (CTFA: OLETH-5) |
| 0.50 g | quaternary ammonium salt of the terpolymer of acrylic |
| | acid/diallyldimethylammonium chloride/acrylamide |
| | (CTFA: POLYQUATERNIUM-39) |
| | Trade name: Merquat. sup. R Plus 3330^{®} |
| 3.00 g | isopropyl alcohol |
| 0.20 g | ethylene diamine tetra acetate (EDTA) |
| 0.50 g | perfume oil |
| ad 100.00 g | water |

### Component B

| | |
|---|---|
| 100.00 g | N-(2'-Hydroxypropyl)-2-mercaptoacetamide |

For the use of the dual-component permanent waving composition, 60g of component A is mixed with 14g of component B to make a hair waving composition that is ready to use. The mixing results in a product with a pH of 6.5.

Normal hair, not previously damaged, is washed, dried with a towel and wound onto curlers with a diameter of 6 mm. Thereafter the hair is moistened thoroughly and evenly with the above-described hair waving composition. After an action time of 15 to 25 minutes, the hair is rinsed thoroughly with water and then oxidatively post-treated with 80g of a 3% aqueous hydrogen peroxide solution. After the curlers are removed, the hair is rinsed again with water, set for a water wave, and then dried. The thus-treated hair has a uniform, lively curliness and offers and excellently formed, stable permanent wave.

### Example 13 Acid Permanent Waving Composition for Normal Hair

| | |
|---|---|
| 7.70 g | N-propyl-2-mercaptoacetamide |
| 22.00 g | N-(3'-hydroxypropyl)-2-mercaptoacetamide |
| 0.50 g | 2,2'-Dithiobis(N-propyl-acetamide) |
| 1.30 g | 2,2'-Dithiobis[N-(3-hydroxypropyl)-acetamide] |
| 5.00 g | isopropyl alcohol |
| 1.00 g | 1,2-propanediol |
| 0.50 g | coconut oil alcohol, ethoxylated with 10 Mol ethylene oxide |
| 0.20 g | Celquat^{®} SC 240 C (National Starch) (CTFA: CELLULOSE KC) |
| 0.30 g | Plex^{®} 7525L (CTFA: POLYQUATERNIUM-35) |
| 0.10 g | vinylpyrrolidone/styrene copolymer |
| | (Antara.sup.R ^{®}430, GAF Corp., New York) |
| 0.50 g | perfume oil |
| ad 100.00 g | water |

The pH of this composition is adjusted with phosphoric acid to 4.5.

Normal hair, not previously damaged, is washed and than dried with a towel. The hair is wound onto curlers with a diameter of 6 mm. After that the hair is moistened thoroughly and evenly with the above-described hair waving composition. After an action time of 25 minutes the hair is rinsed thoroughly with water and then oxidatively post-treated with 80g of a 3% aqueous hydrogen peroxide solution. When the curlers are removed, the hair is rinsed again with water, set for a water wave, and then dried. The thus-treated hair is uniformly curly over the entire length of the hair, which is comparable to the curliness attained by treatment with mildly alkaline permanent waving compositions.

### Example 14 Permanent Waving Composition for Dyed Hair

| | |
|---|---|
| 12.40 g | N-(3'-hydroxypropyl)-2-mercaptoacetamide |
| 1.00 g | Merquat 550 L (CTFA: POLYQUATERNIUM-7) |
| 0.60 g | CTFA: QUATERNIUM-75 |
| 0.80 g | ammonia, 25% aqueous solution |
| 2.00 g | ammonium hydrogen carbonate |
| 2.00 g | dipropyleneglycol monomethyl ester |
| 1.00 g | 1,2-propylene glycol |
| 2.50 g | phosphoric acid ester of decylalcohol ethoxylated with 4 moles of ethylene oxide (CTFA: DECETH-4 PHOSPHATE) |
| 0.30 g | perfume oil |
| ad 100.00 g | water |

The pH of this composition is adjusted with ammonia to 7.3.

### Example 15 Permanent Waving Composition for Dyed Hair

| | |
|---|---|
| 12.40 g | N-(3'-hydroxypropyl)-2-mercaptoacetamide |
| 0.50 g | Merquat 550 L (CTFA: POLYQUATERNIUM-7) |
| 0.30 g | CTFA: METHOXY PEG/PPG-7/3 AMINOPROPYL |
| | DIMETHICONE |
| 0.20 g | CTFA: DIMETHICONE PEG-7 PHOSPHATE |
| 0.60 g | CTFA: QUATERNIUM-75 |
| 0.80 g | ammonia, 25% aqueous solution |
| 2.00 g | ammonium hydrogen carbonate |
| 2.00 g | dipropyleneglycol monomethyl ester |
| 1.00 g | 1,2-propylene glycol |
| 2.50 g | phosphoric acid ester of decylalcohol ethoxylated with 4 moles of ethylene oxide (CTFA: DECETH-4 PHOSPHATE) |
| 0.30 g | perfume oil |
| ad 100.00 g | water |

The pH of this composition is adjusted with ammonia to 7.3.

Hair previously damaged by coloring treatments is washed with a shampoo, towel-dried, and set on curlers with a diameter of 8 mm. Next, the hair waving composition described above is applied evenly to the hair wound on the curlers. The hair is then covered with a plastic hood and heated for 10 min under a drying hood at a temperature of 45° C. The covering is then removed; the hair is rinsed with water and oxidatively post-treated with 100 g of a 3% aqueous hydrogen peroxide solution. After the curlers are removed, the hair is rinsed again with water, set for a water wave, and then dried.

The result of this treatment is a uniform, elastic and durable waving of the hair.

### Examples 16 to 20 Low Alkaline Permanent Waving Compositions for Colored Hair

| | |
|---|---|
| 5.50 g | N-(3'-hydroxypropyl)-2-mercaptoacetamide |
| 0.50 g | 2,2'-dithio-bis[(N-(3-hydroxypropyl)-acetamide] |
| 0.20 g | ammonia, 25 % aqueous solution |
| 2.60 g | ammonium hydrogen carbonate |
| amount as g defined below | **cationic polymer as defined below¹** |
| 1.00 g | aminofunctional siliconpolymer (CTFA: METHOXY PEG/PPG-7/3 AMINOPROPYL DIMETHICONE) |
| 0.30 g | styrene/vinylpyrrolidon-copolymer (CTFA: STYRENE/VP COPOLYMER) |
| 1.50 g | 1,2-propylenglykol |
| 2.00 g | dipropyleneglykol monoethyl ether |
| 2.00 g | urea |
| 1.00 g | hydrogenated castor oil, ethoxylated wit 40 moles of ethylene oxide (CTFA: PEG-40 HYDROGENATED CASTOR OIL) |
| 1.00 g | Genapol^{®} C 100 (CTFA: COCETH-10) |
| 0.50 g | perfume oil |
| ad 100.00 g | water |

The pH of the composition is adjusted with ammonia to 8.2.

### 1 = The kind of cationic polymer and the amount is given in the table below.

| **Example No** | **Amount** | **Cationic Polymer** |
|---|---|---|
| 16 | 0.80 g | Luviquat^{®} PQ 11 PN = copolymer of vinyl pyrrolidone and dimethylamino ethylmethacrylate methosulfate |
| | | (CTFA: POLYQUATERNIUM-11) |
| 17 | 1.50 g | CELQUAT^{®} H-100 = copolymer of hydroxyethyl cellulose and diallyldimethylammonium chloride |
| | | (CTFA: POLYQUATERNIUM-4) |
| 18 | 1.70 g | MERQUAT^{®} S = copolymer of acrylamide and diallyldimethylammonium chloride |
| | | (CTFA: POLYQUATERNIUM-7) |
| 19 | 0.70 g | GAFQUAT^{®} HSI = copolymer of vinyl pyrrolidone and methacrylamidopropyl trimethyl ammonium chloride |
| | | (CTFA: POLYQUATERNIUM-28) |
| 20 | 1.20 g | MERQUAT^{®} 281 = copolymer of diallyldimethyl ammonium chloride and acrylic acid (80/20) |
| | | (CTFA: POLYQUATERNIUM-22) |

After winding the hair on curlers the above permanent waving agent is uniformly applied onto the curlers. The permanent waving agent is left on the curlers for 12 minutes. An infrared drying hood is used at a temperature of 40°C. During the treating time a much better and more pleasant smell is noticed by the hairdresser as well as by the customer compared to permanent waving agents commonly used. The hair is than rinsed with lukewarm water. Thereafter 80g of a fixing agent of example 1 is applied onto the curler.

The fixing agent is left on the hair for 6 minutes. Finally the hair is again rinsed with lukewarm water and the curlers are removed. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Example 21 to 24

### Low Alkaline Permanent Waving Compositions for Unmanageable Hair

| | |
|---|---|
| 10.00 g | N-(3'-hydroxypropyl)-2-mercaptoacetamide |
| 0.50 g | 2,2'-dithio-bis[(N-(3-hydroxypropyl)-acetamide] |
| 6.00 g | ammonia (25 % aqueous solution) |
| 3.50 g | ammonium hydrogen carbonate |
| 0.20 g | cetyltrimethylammonium chloride |
| amount as g defined below | **cationic polymer as defined below²** |
| 1.00 g | 1,3-butandiol |
| 1.50 g | 1,2-propylenglycol |
| 2.00 g | dipropylenglycol monoethylether |
| 3.50 g | urea |
| 1.50 g | oleic alcohol, ethoxylated with 10 mol of ethylenoxide |
| | (CTFA: OLETH-10) |
| 1.00 g | Genapol^{®} C 100 (CTFA: COCETH-10) |
| 0.60 g | perfume oil |
| ad 100.00 g | water |

The pH of the composition is adjusted with ammonia to 8.9.

### 2 = The kind of cationic polymer and the amount is given in the table below.

| **Example No** | **Amount** | **Cationic Polymer** |
|---|---|---|
| 21 | 0.20 g | Abil Quat^{®} 3474 = dimethylsiloxane, 3-(3-((3-cocoamidopropyl)dimethylammonio)-2-hydroxyprpoxy)propyl group terminated acetate |
| | | (CTFA: QUATERNIUM-80; CAS 134737-05-6) |
| 22 | 1.00 g | MERQUAT^{®} 281 = copolymer of acrylic acid and diallyldimethylammonium chloride |
| | | (CTFA: POLYQUATERNIUM-22) |
| 23 | 0.40 g | LUVIQUAT^{®} HM 552 = copolymer of 1-vinyl-2-pyrrolidone and 3-methyl-1-vinylimidazolium chloride |
| | | (CTFA: POLYQUATERNIUM-16) |
| 24 | 0.18 g | SALCARE^{®}SC 60 = copolymer of acrylamide and acrylamidopropyltrimonium chloride |
| | | (CTFA: ACRYLAMIDOPROPYLTRIMONIUM CHLORIDE/ACRYLAMIDE COPOLYMER) |

After winding the hair on curlers the above permanent waving agent is uniformly applied onto the curlers. The permanent waving agent is left on the curlers for 14 minutes. An infrared drying hood is used at a temperature of 42°C. During the treating time a much better and more pleasant smell is notices by the hairdresser as well as by the customer compared to permanent waving agents commonly used. The hair is than rinsed with lukewarm water. Thereafter 80g of a fixing agent of example 1 is applied onto the curler.

The fixing agent is left on the hair for 7 minutes. Than the curlers are removed and the hair is post neutralized for 5 min with 40g of the fixing agent of example 1. Finally the hair is again rinsed with lukewarm water. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Examples 25 to 30 Alkaline Permanent Waving Compositions for Colored Hair

| | |
|---|---|
| 8.00 g | N-(3'-hydroxypropyl)-2-mercaptoacetamide |
| amount as g defined below | **cationic polymer as defined below³** |
| 2.00 g | Genapol^{®} C 100 (CTFA: COCETH-10) |
| 1.00 g | ammonia, 25 % aqueous solution |
| 2.00 g | ammonium hydrogen carbonate |
| 0.01 g | (CTFA: SIMETHICONE) |
| 0.13 g | hydroxethylcellulose |
| 0.30 g | perfume oil |
| ad 100.00 g | water |

The pH of the composition is adjusted with ammonia to 8.1.

### 3 = The kind of cationic polymer and the amount is given in the table below.

| **Example No** | **Amount** | **Cationic Polymer** |
|---|---|---|
| 25 | 1.10 g | Abil Quat^{®} 3272 = dimethylsiloxane, 3-(3-((3-cocoamidopropyl)dimethylammonio)-2-hydroxyprpoxy)propyl group terminated acetate |
| | | (CTFA: QUATERNIUM-80; CAS 134737-05-6) |
| 26 | 0.80 g | Luviquat^{®} PQ 11 PN = copolymer of vinyl pyrrolidone and dimethylamino ethylmethacrylate methosulfate |
| | | (CTFA: POLYQUATERNIUM-11) |
| 27 | 1.50 g | CELQUAT^{®} H-100 = copolymer of hydroxyethyl cellulose and diallyldimethylammonium chloride |
| | | (CTFA: POLYQUATERNIUM-4) |
| 28 | 1.70 g | MERQUAT^{®} S = copolymer of acrylamide and diallyldimethylammonium chloride |
| | | (CTFA: POLYQUATERNIUM-7) |
| 29 | 0.70 g | GAFQUAT^{®} HSI = copolymer of vinyl pyrrolidone and methacrylamidopropyl trimethyl ammonium chloride |
| | | (CTFA: POLYQUATERNIUM-28) |
| 30 | 1.20 g | MERQUAT^{®} 281 = copolymer of diallyldimethyl ammonium chloride and acrylic acid (80/20) |
| | | (CTFA: POLYQUATERNIUM-22) |

After winding the hair on curlers the above permanent waving agent is uniformly applied onto the curlers. The permanent waving agent is left on the curlers for 12 minutes. An infrared drying hood is used at a temperature of 40°C. During the treating time a much better and more pleasant smell is noticed by the hairdresser as well as by the customer compared to permanent waving agents commonly used. The hair is than rinsed with lukewarm water. Thereafter 80g of a fixing agent of example 1 is applied onto the curler.

The fixing agent is left on the hair for 6 minutes. Finally the hair is again rinsed with lukewarm water and the curlers are removed. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Example 31 Hair Straightening Cream for Dyed Hair

| | |
|---|---|
| 2.00 g | N-propyl-2-mercaptoacetamide |
| 2.00 g | N-(3'-hydroxypropyl)-2-mercaptoacetamide |
| | (90 % aqueous solution) |
| 4.00 g | ammonium thioglycolate (70 % aqueous solution) |
| 3.00 g | ammonia, 25% aqueous solution |
| 0.50 g | Abilsoft^{®} AF 100 (CTFA: Methoxy PEG/PPG-7/3 Aminopro- |
| | pyldimethicone) |
| 1.00 g | Dimethylsiloxane, 3-(3-((3-cocoamidopropyl)dimethylammonio)-2-hydroxyprpoxy)propyl groupterminated acetate; |
| | (Abil Quat^{®} 3474) |
| | (CTFA: QUATERNIUM-80; CAS 134737-05-6) |
| 1.00 g | sodium laurylsulfate |
| 9.00 g | cetylstearyl alcohol (Lanette^{®} O) |
| 1.80 g | cetylstearyl alcohol polyethylenglycol (25) ether |
| | (Cremophor^{®} A 25) |
| 2.80 g | vaseline (CTFA: PETROLATUM) |
| 0.50 g | perfume oil |
| ad 100.00 g | water |

The pH of this composition is adjusted with ammonia to 8.2

The above hair straightening cream is applied onto the dry hair by a brush. Thereafter the hair is combed several times to smoothen said hair. After the treating time of 15 minutes at a temperature of 25° C the hair is rinsed with water and than oxidatively post-treated with 90g of a fixing composition. After the treating time of 8 min the hair is rinsed again with water. During the treating time a much better and more pleasant smell is notices by the hairdresser as well as by the customer compared to permanent straightening compositions commonly used. The hair exhibits a uniform straight shape from the tips to the roots and the hair tips show a good structure. The hair has a good touch and fine wet combing properties. During or after the hair treating procedure described above neither irritations of the skin nor other effects on the skin were realized.

### Example 32 Hair Straightening Cream for Very Curly Hair

| | |
|---|---|
| 14.00 g | N-(3'-hydroxypropyl)-2-mercaptoacetamide |
| | (90 % aqueous solution) |
| 5.00 g | monoethanolamine thioglycolate (70 % aqueous solution) |
| 3.00 g | monoethanolamine |
| 1.00 g | sodium laurylsulfate |
| 11.00 g | cetylstearyl alcohol |
| 2.50 g | cetylstearyl alcohol polyethylenglycol (20) ether |
| 3.20 g | Vaseline (CTFA: PETROLATUM) |
| 0.50 g | aminoethylaminopropylsiloxane/dimethylsiloxan-copolymer emulsion; GE Toshiba Silicone (CTFA: AMODIMETHICONE) |
| 0.50 g | perfume oil |
| ad 100.00 g | water |

The pH of this composition is adjusted with ammonia (25 % aqueous solution) to 9.3

The above hair straightening cream is applied onto the dry hair with a brush. After the treating time of 16 minutes at a temperature of 28°C the hair is rinsed with water. Thereafter the hair is ironed with a hot iron and than oxidatively post-treated with 80g of a fixing composition. During the treating time a much better and more pleasant smell is notices by the hairdresser as well as by the customer compared to commonly used permanent straightening agents. Thereafter the hair is combed several times to smoothen said hair. After the treating time of 7 min the hair is rinsed again with water. The hair exhibits a uniform straight shape from the tips to the roots and the hair tips show a good structure. The hair has a good touch and fine wet combing properties. During or after the hair treating procedure described above neither irritations of the skin nor other effects on the skin were realized.

### Example 33 Permanent Waving Composition for Normal Hair

| | |
|---|---|
| 7.70 g | N-propyl-2-mercaptoacetamide |
| 8.70 g | N-(3'-hydroxypropyl)-2-mercaptoacetamide |
| 5.00 g | isopropyl alcohol |
| 1.00 g | 1,2-propanediol |
| 0.50 g | Coconut oil alcohol, ethoxylated with 10 Mol ethylene oxide |
| 0.50 g | Aminoethylaminopropylsiloxane/dimethylsiloxan-copolymer emulsion; GE Toshiba Silicone (CTFA: AMODIMETHICONE) |
| 0.10 g | vinylpyrrolidone/styrene copolymer |
| | (Antara.sup.R 430, GAF Corp., New York) |
| 0.50 g | perfume oil |
| ad 100.00 g | water |

The pH of this composition is adjusted with phosphoric acid (85 % aqueous solution) to 4.5.

Normal hair, not previously damaged, is washed and than dried with a towel. After winding the hair on curlers the above permanent waving agent is uniformly applied onto the curlers. The permanent waving agent is left on the curlers for 14 minutes. An infrared drying hood is used at a temperature of 42°C. During the treating time a much better and more pleasant smell is notices by the hairdresser as well as by the customer compared to commonly used permanent waving agents. The hair is than rinsed with lukewarm water. Thereafter 80g of a fixing agent of example 1 is applied onto the curler. The fixing agent is left on the hair for 7 minutes. Finally the hair is again rinsed with lukewarm water and the curlers are removed. If necessary the hair is post neutralized with 40 ml fixing agent for 5 min and is then rinsed again with lukewarm water. The hair, even at the hair tips, shows in dry and wet condition a well groomed look, a good touch and fine wet combing properties. It exhibits a good elasticity from the tips to the roots. Even after 3 days the hair keeps its pleasant smell. During or after the hair treating procedure described above neither irritations of the skin nor other effects on the skin were realized.

### Example 34 Composition and Process for Waving and Dyeing Hair

Natural hair is first colored with the oxidative hair colorant of component A1. The component is left on for 20 min. The hair is then neutralized with an acidic intermediate treatment agent (component B1). After an action period of 3 min, the damp hair is rolled onto curlers with a diameter of 8 mm, and the wave agent (component C1) is applied and allowed to work for 20 min. The wave agent is then rinsed out with water, the hair is fixed with a fixing agent (component D1) for 10 min, and it is then treated with an acidic agent.

### Oxidative hair colorant (component A1)

### Component (1)

| | |
|---|---|
| 1.00 g | castor oil, oxyethylated with 35 mol ethylene oxide |
| 0.20 g | sodium sulfite |
| 8.10 g | ammonia, 25% aqueous solution |
| 0.10 g | 3-aminophenol |
| 0.04 g | amino-4-[(2-hydroxyethyl)amino]anisole |
| 0.60 g | resorcinol |
| 0.40 g | p-toluene diamine sulfate |
| 0.03 g | 2-amino-5-methylphenol |
| 0.50 g | 4-amino-3-methylphenol |
| 0.30 g | quaternized hydroxy ethyl cellulose |
| | (POLYQUATERNIUM-10) |
| 2.00 g | ethanol |
| ad 100.00 g | water |

### Component (2)

| | |
|---|---|
| 12.00 g | hydrogen peroxide, 50% |
| 0.10 g | salicylic acid |
| 0.20 g | disodium hydrogen phosphate |
| 0.15 g | o-phosphoric acid |
| 1.00 g | castor oil, oxyethylated with 35 mol ethylene oxide |
| 0.10 g | vinylpyrrolidone/styrene copolymer |
| 0.10 g | perfume oil |
| ad 100.00 g | water |

Before use, 40 g of the liquid color vehicle (component (1)) is mixed with 40 g of the hydrogen peroxide solution (component (2)). The pH value of the ready-to-use agent obtained (component A1) is 9.8. 120 g of the ready-to-use agent is applied to gray, human hair and the mixture is left in for 20 min at room temperature.

### Intermediate treatment agent (component B1)

| | |
|---|---|
| 2.0 g | mixture of cetyl and stearyl alcohols |
| 1.0 g | Vaseline |
| 1.0 g | citric acid |
| 0.5 g | perfume oil |
| ad 100.00 g | water |

### Permanent waving agent (Component C1)

| | |
|---|---|
| 9.00 g | N-(3'-hydroxypropyl)-2-mercaptoacetamide |
| 6.00 g | 2,2'-dithio-bis[(N-(3-hydroxypropyl)-acetamide] |
| 1.00 g | ammonia, 25% aqueous solution |
| 4.00 g | ammonium hydrogen carbonate |
| 1.00 g | castor oil, oxyethylated with 35 mol ethylene oxide |
| 1.00 g | copolymer of hydroxyethyl cellulose and diallyldimethylammonium chloride (CTFA: POLYQUATERNIUM-4) |
| 0.50 g | perfume oil |
| ad 100.00 g | water |

The acidic intermediate treatment agent (Component B1) has a pH value of 3.2 and neutralizes the alkaline component A1 on the curlers. Subsequently, the alkaline permanent wave lotion (component C1) is evenly distributed to the hair on the curlers. The solution is then left on the hair for 20 min, and a hood-type infrared radiator is used at a temperature of 40° C (104 °F). During the treating time a much better and more pleasant smell is notices by the hairdresser as well as by the customer compared to commonly used permanent waving agents. The rolled up hair is then treated with 80 g of the following fixing agent.

### Liquid fixing agent (component D1)

| | |
|---|---|
| 4.00 g | hydrogen peroxide, 50% |
| 0.10 g | salicylic acid |
| 0.20 g | disodium hydrogen phosphate |
| 0.15 g | o-phosphoric acid |
| 1.00 g | castor oil, oxyethylated with 35 mol ethylene oxide |
| 0.10 g | vinylpyrrolidone/styrene-mixed polymer |
| 0.10 g | perfume oil |
| ad 100 g | water |

There is then an action period of 10 minutes. After the action period has elapsed, the curlers are removed and the hair is thoroughly rinsed with warm water and then treated with a known acidic rinse. The hairstyle is then created in the normal manner with a blow dryer and brush. The hair is colored to a warm brown tone and exhibits a beautiful shine.

The hair exhibits a good elasticity from the tips to the roots. Even after 3 days the hair keeps its pleasant smell. No fading of the color or bleeding out of the dye could be noticed during a hair wash. During or after the hair treating procedure described above neither irritations of the skin nor other effects on the skin were realized.

### Example 35 Composition and Process for Waving and Dyeing Damaged Hair

Hair damaged by oxidation is first colored with the oxidative hair colorant of Component A2. The component is left on for 20 min. The hair is then neutralized with an acidic intermediate treatment agent (Component B2). After an action period of 5 minutes, the hair is rolled onto curlers with a diameter of 10 mm, and the wave agent (Component C2) is applied and allowed to work for 10 min. The wave agent is then rinsed out with water, the hair is fixed, and it is then treated with an acidic material.

### Oxidative hair colorant (Component A2)

| Component (a) | Liquid color vehicle |
|---|---|
| 2.80 g | 2,5-diamino toluene sulfate |
| 1.00 g | resorcinol |
| 0.40 g | m-aminophenol |
| 0.20 g | 2-amino-4-(2'-hydroxyethylamino)anisole sulfate |
| 5.00 g | lauryl glucoside (Plantaren^{®} 1200 sold by Henkel KGaA, Germany) |
| 5.00 g | sodium lauryl alcohol diglycol ether sulfate, 28 % aqueous solution |
| 0.10 g | valine |
| 0.30 g | ascorbic acid |
| 0.10 g | ethylene diamine tetraacetic acid |
| 0.10 g | keratin hydrolysate |
| 12.20 g | ammonia, 25% aqueous solution |
| 16.00 g | propylene glycol |
| ad 100.00 g | water |

| Component (b): | Hydrogen Peroxide Emulsion |
|---|---|
| 10.00 g | cetylstearyl alcohol |
| 3.00 g | acrylates/Ceteth-20 Itaconate Copolymer |
| | (Structure 3001^{®} sold by the National Starch & Chemical Company, USA) |
| 5.00 g | polyoxypropylene oxide(1)polyoxyethylenoxide(9) |
| | laurylether (CTFA: PPG-1-PEG-9 LAURYL ETHER) |
| 4.00 g | sodium lauryl alcohol diglycol ether sulfate, 28 % aqueous solution |
| 20.00 g | hydrogen peroxide, 35 % aqueous solution |
| 0.30 g | perfume |
| ad 100.00 g | water |

Before use, 40 g of the liquid color vehicle (Component (a)) is mixed with 80 g of the hydrogen peroxide emulsion (Component (b)). The pH value of the ready-to-use agent obtained (Component A2)is 10.0. 120 g of the ready-to-use agent is applied to gray, human hair and the mixture is left in for 20 minutes at room temperature.

### Intermediate treatment agent (Component B2)

| | |
|---|---|
| 2.00 g | cetyltrimethylammonium chloride |
| 8.00 g | citric acid |
| 10.00 g | lactic acid, 90% aqueous solution |
| 5.00 g | glyoxylic acid |
| ad 100 g | water |

### Permanent restructuring agent (Component C2)

| | |
|---|---|
| 14.50 g | N-propyl-2-mercaptoacetamide |
| 0.20 g | 2,2'-dithio-bis[(N-(3-hydroxypropyl)-acetamide] |
| 0.50 g | Merquat 550 L (CTFA: Polyquaternium-22) |
| 0.60 g | ammonia, 25% aqueous solution |
| 3.00 g | ammonium hydrogen carbonate |
| 2.00 g | castor oil, oxyethylated with 35 mol ethylene oxide |
| 0.50 g | perfume oil |
| ad 100.00 g | water |

The acidic intermediate treatment (Component B2) has a pH value of 3.0 and thus neutralizes the alkaline component A2. After the action period of 5 minutes, the permanent wave solution (Component C2) is evenly distributed to the rolled up hair. The solution is then left on the hair for 10 minutes, and a hood-type infrared radiator is used at a temperature of 40°C (104 °F). During the treating time a much better and more pleasant smell is notices by the hairdresser as well as by the customer compared to commonly used permanent waving agents. The rolled up hair is then treated with 80 g of the following fixing agent (Component D2).

### Viscous fixing agent (Component D2)

| | |
|---|---|
| 4.00 g | cetyl stearyl alcohol |
| 0.10 g | vinylpyrrolidone/dimethylaminoethyl methacrylate methosulfate copolymer |
| 0.10 g | salicylic acid |
| 0.30 g | perfume oil |
| ad 100.00 g | water |

There is then an action period of 15 minutes. The curlers are subsequently removed, and the hair is thoroughly rinsed with warm water. The hairstyle is then created in the normal manner with a blow dryer and brush.

The hair has taken on an even, dark brown tone, exhibits a smooth, pleasant hold, and has a beautiful shine. Even after 3 days the hair keeps its pleasant smell. During or after the hair treating procedure described above neither irritations of the skin nor other effects on the skin were realized. No fading of the color or bleeding out of the dye could be noticed during a hair wash.

## Claims

1. A composition for permanent hair shaping comprising
(i) 0.5 to 35 % by weight of a N-alkyl-2-mercaptoacetamide of formula 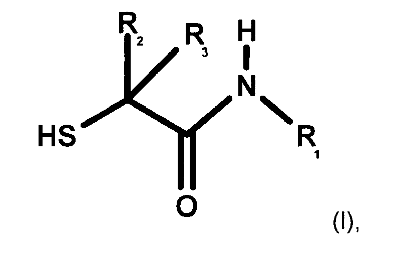 wherein R1 is a straight chain alkyl residue with 3 to 6 carbon atoms or a straight chain hydroxyalkyl with 3 to 6 carbon atoms, R2 and R3 are, independently from each other, hydrogen or straight chain alkyl residues with 1 to 3 carbon atoms, or the salt thereof,
(ii) 0.1 to 15 % by weight of at least one cationic polymer selected from the groups consisting of cationic copolymers, cationic polysaccharides, cationic protein hydrolysates and cationic silicone polymers; and
(iii) 5 to 95 % by weight of water.

2. Composition according to claim 1, wherein the N-alkyl-2-mercapto-acetamide of formula (I) is selected from N-propyl-2-mercaptoacetamide, N-(2'-hydroxypropyl)-2-mercaptoacetamide and N-(3'-hydroxypropyl)-2-mercaptoacetamide.

3. Composition according to claim 1 or 2, wherein the N-alkyl-2-mercaptoacetamide of formula (I) is present in an amount of from 4 to 20% by weight.

4. Composition as recited in any one of claims 1 to 3, comprising another hair keratin-reducing agent, which is selected from thioglycolic acid, cysteine and thiolactic acid, or the salt thereof.

5. Composition as recited in any one of claims 1 to 4, wherein the total amount of the hair keratin-reducing agents in the permanent shaping composition is from 2% to 28 % by weight.

6. Composition as recited in any one of claims 1 to 5, wherein the total amount of the cationic polymer in the permanent shaping composition is from about 0.2% to about 3% by weight of the composition.

7. Composition as recited in any one of claims 1 to 6, wherein the permanent shaping composition comprises a disulfide of a hair keratin-reducing agent.

8. Composition as recited in claim 7, wherein the disulfide of the hair keratin-reducing agent is selected from 2,2'-dithio-bis[N-(3-hydroxypropyl)-acetamide], 2,2'-dithio-bis[N-(2-hydroxypropyl)-acetamide], 2,2'-dithio-bis(propyl)-acetamide and dithioglycolic acid or the salt thereof.

9. Composition as recited in one of claims 7 or 8, wherein the disulfide of the hair keratin-reducing agent is contained in a quantity of from 0.5% to 20 % by weight.

10. Composition as recited in any one of claims 7 to 9, wherein the ratio of the hair keratin-reducing agent or the mixture thereof to the disulfide of a hair keratin-reducing agent is from 6 : 1 to 1 : 1.

11. Composition according to anyone of claims 1 to 10, wherein the pH-value is from 4 to 10.

12. Composition according to anyone of claims 1 to 11, wherein the cationic polymer is selected from the group of: the copolymer of hydroxyethyl cellulose and diallyldimethylammonium chloride, the copolymer of acrylamide and diallyldimethylammonium chloride, the copolymer of vinyl pyrrolidone and dimethylamino ethylmethacrylate methosulfate, the copolymer of acrylamide and betamethacrylyloxyethyl trimethyl ammonium chloride, the copolymer of polyvinyl pyrrolidone and imidazolimine methochloride, the copolymer of diallyldimethyl ammonium chloride and acrylic acid, the copolymer of vinyl pyrrolidone and methacrylamidopropyl trimethyl ammonium chloride, the methosulfate of the copolymer of methacryloyloxyethyl trimethylammonium and methacryloyloxyethyl dimethylacetylammonium, quaternized hydroxyethyl cellulose; dimethylsiloxane 3-(3-((3-cocoamidopropyl)dimethylammonio)-2-hydroxyprpoxy)propyl groupterminated acetate; the copolymer of aminoethylaminopropylsiloxane and dimethylsiloxan; the polyethylene glycol derivative of aminoethylaminopropylsiloxane/dimethylsiloxan-copolymer and cationic silicone polymers.

13. A method for permanent shaping of hair, said method comprising the steps of:
(a) putting the hair in a desired shape;
(b) before and/or after the hair is put in the desired shape, applying an amount of a permanent shaping composition to the hair and allowing the permanent shaping composition to act on the hair for a predetermined acting time sufficient for the permanent shaping of the hair;
(c) subsequently rinsing the hair with water or applying an intermediate treatment agent,
(d) performing an oxidative post-treatment of the hair;
(e) after the oxidative post-treatment, rinsing the hair with water and drying the hair, wherein said permanent shaping composition is a composition according to anyone of claims 1 to 12.

14. Method as recited in claim 13, wherein the intermediate treatment agent contains betaine, citric acid, lactic acid, and/or glyoxylic acid.

15. Method as recited in one of claims 13 or 14, wherein the intermediate treatment agent has a pH of from 2 to 4.

16. Method as recited in one of claims 13 to 15, wherein the intermediate treatment agent has an action period of from 3 to 5 minutes.

17. The method recited in any one of claims 13 to 16, further comprising allowing said composition for permanent shaping of hair to act on the hair for from 5 to 20 minutes prior to said rinsing of the hair with water.

18. The method recited in any one of claims 13 to 17, wherein said amount of said at least one N-alkyl-2-mercaptoacetamide of formula (I) is from 50 to 120 g.

19. The method as defined in claim 13, further comprising putting the hair in a water wave after having oxidatively post-treated the hair.
